Europäisches Patentamt

European Patent Office

Office européen des brevets

(11) Numéro de publication: **0 416 975 A1**

## (12) DEMANDE DE BREVET EUROPEEN

(21) Numéro de dépôt: **90402339.7**

(22) Date de dépôt: **22.08.90**

(51) Int. Cl.⁵: **A61M 5/315**, G01F 11/02

(30) Priorité: **25.08.89 FR 8911406**

(43) Date de publication de la demande:
**13.03.91 Bulletin 91/11**

(84) Etats contractants désignés:
**AT BE CH DE DK ES FR GB GR IT LI LU NL SE**

(71) Demandeur: **Taddei, André**
**32, rue Centrale**
**F-06300 Nice(FR)**

(72) Inventeur: **Taddei, André**
**32, rue Centrale**
**F-06300 Nice(FR)**

(74) Mandataire: **Plaçais, Jean-Yves et al**
**Cabinet Netter, 40, rue Vignon**
**F-75009 Paris(FR)**

(54) **Dispositif portatif pour effectuer des dosages multiples d'un produit liquide ou pâteux.**

(57) L'invention concerne un dispositif de poche, pour effectuer, à partir d'une unique recharge (4), plusieurs injections successives exactement dosées. Dans le corps (2), se trouve un dispositif pour pousser vers le bas (flèche 10), le piston de la recharge (4) avec, d'une part, un système de butée pour régler exactement la course du piston, d'autre part, un mécanisme à ailettes élastiques anti-retour du piston pour la sécurité.

Application : traitement à l'héparine ou à l'insuline, même pour un utilisateur aveugle.

FIG.3

EP 0 416 975 A1

# DISPOSITIF PORTATIF POUR EFFECTUER DES DOSAGES MULTIPLES D'UN PRODUIT LIQUIDE OU PÂTEUX

La présente invention est relative à un dispositif portatif d'un type nouveau, destiné à contenir une charge d'un produit liquide ou pâteux, pour permettre ensuite de le délivrer par des doses successives dont chacune est à volume bien déterminé. De plus, l'utilisateur doit pouvoir régler à volonté et avec précision le volume de la dose, ce volume pouvant varier d'une dose à l'autre.

Un tel dispositif susceptible de fournir des dosages successifs trouve son application dans des domaines variés. Par exemple, on peut l'utiliser pour délivrer des doses successives de colle, d'un solvant, d'un médicament. Dans le domaine des applications médicales, l'invention vise plus particulièrement, quoique non exclusivement, le dosage et les injections de substances telles que l'héparine ou l'insuline.

On sait que l'insuline se présente sous la forme d'un liquide utilisé par injections successives dans le corps humain, pour le traitement du diabète. L'utilisateur diabétique est amené à se piquer lui-même plusieurs fois par jour pour s'administrer des doses variables d'insuline, par la technique de l'injection sous-cutanée. Pour lui, le problème est donc de disposer d'un appareil portatif, lui permettant, à tout moment et en tout lieu, de mesurer avec précision le volume de la dose d'insuline à administrer ensuite par piqûre.

Actuellement, ce problème est mal résolu. La plupart du temps, l'utilisateur utilise une seringue hypodermique graduée, dans laquelle il place une carpule ou recharge, puis il procède à l'injection en enfonçant le poussoir d'un piston dont le déplacement défile devant une graduation. Les risques d'erreurs sont nombreux en ce qui concerne la précision du dosage. De plus, le nombre et la nature des manipulations nécessaires à chaque fois ne permettent pas d'assurer une bonne aseptie. En définitive, l'utilisateur n'a pas la possibilité d'effectuer correctement plusieurs injections dosées à partir d'une seule carpule.

La présente invention a pour but d'éviter ces inconvénients en réalisant un dispositif susceptible de permettre, sans risque d'erreurs, d'effectuer un dosage précis, même pour de très petites quantités, du volume de la dose délivrée à chaque opération. De plus, il vise à permettre la manipulation suivant un processus simple, ne compromettant pas l'aseptie.

Le dispositif portatif de délivrance de doses multiples contrôlées comprend :
- un corps creux allongé propre à loger intérieurement une recharge d'un produit liquide, actionnée par poussée, ainsi qu'à recevoir du côté aval de la recharge un embout de délivrance, tel qu'une aiguille creuse d'injection,
- un poussoir monté à coulissement dans le corps, du côté amont de la recharge,
- des moyens limiteurs de l'excursion permise au poussoir vers la recharge, et
- un moyen anti-retour constitué d'au moins une ailette flexible agissant au niveau du poussoir et du corps.

Suivant une caractéristique de l'invention, le poussoir comprend une tige de poussée, la ou les ailettes formant anti-retour vers l'amont pour la tige de poussée par rapport au corps, un manchon coopère avec la tige de poussée également par l'intermédiaire d'au moins une ailette, formant anti- retour aval. La ou les ailettes sont prévues suivant une disposition qui les fait converger vers le centre en direction de l'embout du dispositif; elles coopèrent de préférence avec des crans ou des sections de filetage, qui peuvent être des sections de filetage périphérique du poussoir.

Avantageusement, il est prévu un rappel élastique du manchon vers l'amont.

Le dessin annexé, donné à titre d'exemple non limitatif, permettra de mieux comprendre et de développer les caractéristiques de l'invention, et les avantages qu'elle est susceptible de procurer.

La figure 1 est une coupe longitudinale d'un dispositif selon l'invention, réalisé à la façon d'un stylo, que l'utilisateur peut porter dans sa poche, cette coupe montrant l'appareil à vide.

La figure 2 est une coupe longitudinale analogue, de l'appareil chargé et prêt à l'emploi.

La figure 3 est une vue éclatée, correspondant à la figure 1.

La figure 4 est une vue éclatée d'un autre mode de réalisation selon l'invention.

La figure 5 est une coupe longitudinale partielle, correspondant à une variante de la figure 4.

La figure 6 est une coupe analogue du corps et du poussoir.

La figure 7 montre une réalisation possible de la bague de butée.

La figure 8 est un détail en coupe montrant l'extrémité du poussoir et sa liaison avec le piston refoulant le liquide dans le carpule.

La figure 9 est une coupe longitudinale du capuchon supérieur.

La figure 10 est une vue suivant la direction de la flèche X (figure 9), montrant la disposition des ailettes anti-retour.

Les figures 11 à 13 montrent, en coupe transversale, le fonctionnement du cliquet intérieur de la bague de butée lorsqu'on procède à un dosage.

Les figures 14 à 16 sont des vues analogues pour une autre variante où le cliquet est à l'extérieur de la bague.

Les figures 17 à 19 sont des vues analogues, pour une variante supplémentaire, où la bague comporte extérieurement un doigt d'arrêt à rappel élastique.

La figure 20 est une vue en perspective d'une autre variante.

Les figures 21 à 23 sont des coupes transversales, montrant le cliquet du système de dosage selon la figure 20.

Les figures 24 et 25 sont des coupes analogues, suivant XXIV-XXIV (figure 20).

Les figures 26 et 27 sont des coupes suivant XXVI-XXVI (figure 20).

Les figures 28 à 31 sont des coupes longitudinales de la partie supérieure du dispositif réalisé suivant des variantes à ressort de rappel.

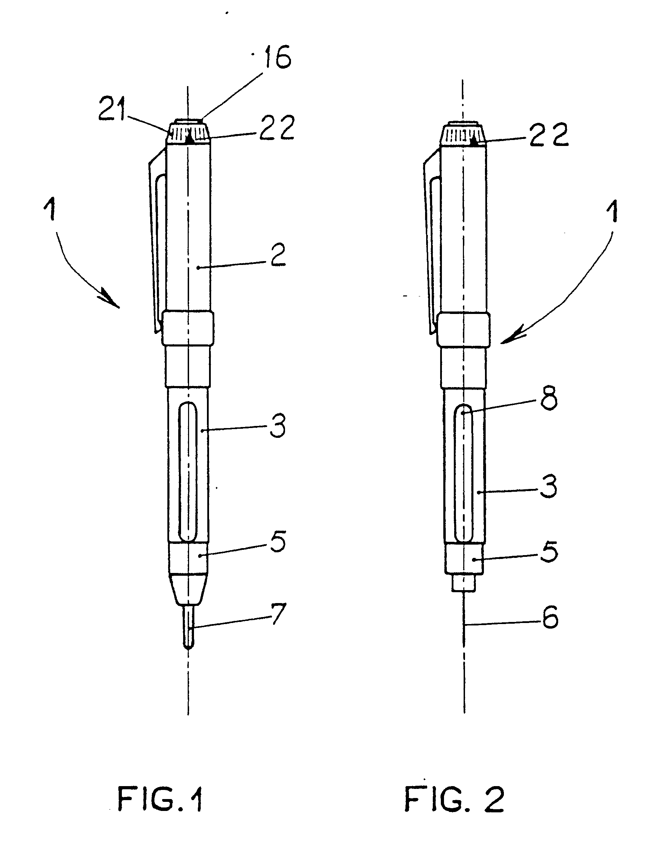

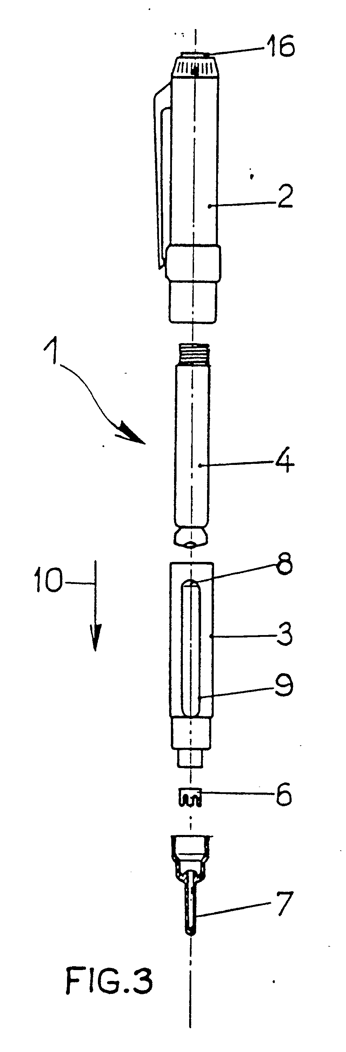

On a représenté sur les figures 1 à 3 un stylo 1 selon l'invention, particulièrement adapté pour effectuer des injections d'héparine ou d'insuline. Il comprend :

- un corps 2, dans lequel se trouve le mécanisme de dosage qui sera décrit plus loin;

- une enveloppe 3, fixée de façon amovible au corps 2, et susceptible de recevoir une cartouche de recharge ou une carpule 4, contenant le produit à injecter;

- un embout 5, équipant l'extrémité de l'enveloppe 3, pour recevoir, à la façon habituelle, une aiguille creuse 6, pour piqûres hypodermiques, et son capuchon protecteur 7.

Bien entendu, l'embout 5 et ses accessoires 6,7, peuvent être supprimés ou modifiés selon la nature du produit à doser de la cartouche 4 : il reste bien entendu que ce produit peut être aussi bien un produit pharmaceutique que de la colle, de la résine, ou toute autre substance.

S'il s'agit d'une carpule 4 pour médicament, on sait qu'elle contient un piston 8. La carpule 4 ayant des parois transparentes, on prévoira de préférence dans l'enveloppe 3 une ouverture longitudinale 9, permettant de surveiller visuellement la position et la progression du piston 8 qui s'enfonce progressivement suivant la direction de la flèche 10, au fur et à mesure des injections successives.

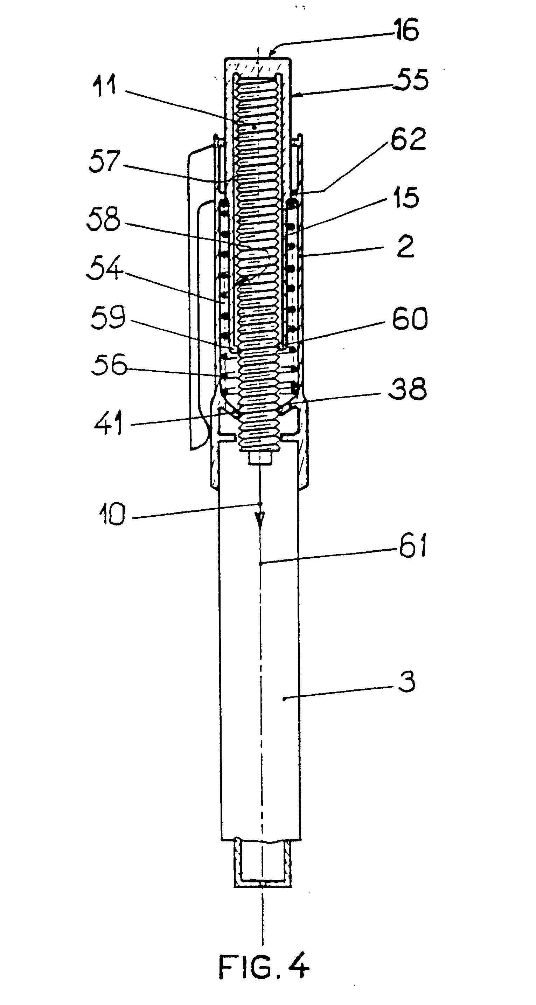

Le mécanisme de dosage (figures 4 à 10) comprend :

- un poussoir 11, qui se présente sous la forme d'une tige filetée extérieurement pourvue, à son extrémité aval, d'un taquet 12, susceptible de venir s'adapter contre le piston 8;

- une bague de butée 13, vissée à la façon d'un écrou réglable sur le poussoir fileté 11;

- une face de butée transversale fixe 14, solidaire du corps fixe 2.

Dans l'exemple illustré sur les figures 1 à 4, la bague de butée 13 est solidaire d'un manchon poussoir 15, qui coiffe le sommet du poussoir fileté 10, et se termine à son sommet, par un bouchon 16. Ce dernier dépasse au sommet du stylo, et il constitue le bouton poussoir qu'actionnera l'utilisateur.

Le poussoir fileté 11 est pourvu de moyens anti-rotation, constitués par au moins une rainure longitudinale 17, creusée le long de la tige filetée. Ces rainures sont, par exemple, au nombre de quatre. Dans chaque rainure 17 peut coulisser un ergot fixe 18 qui dépasse radialement, à l'intérieur du corps 2.

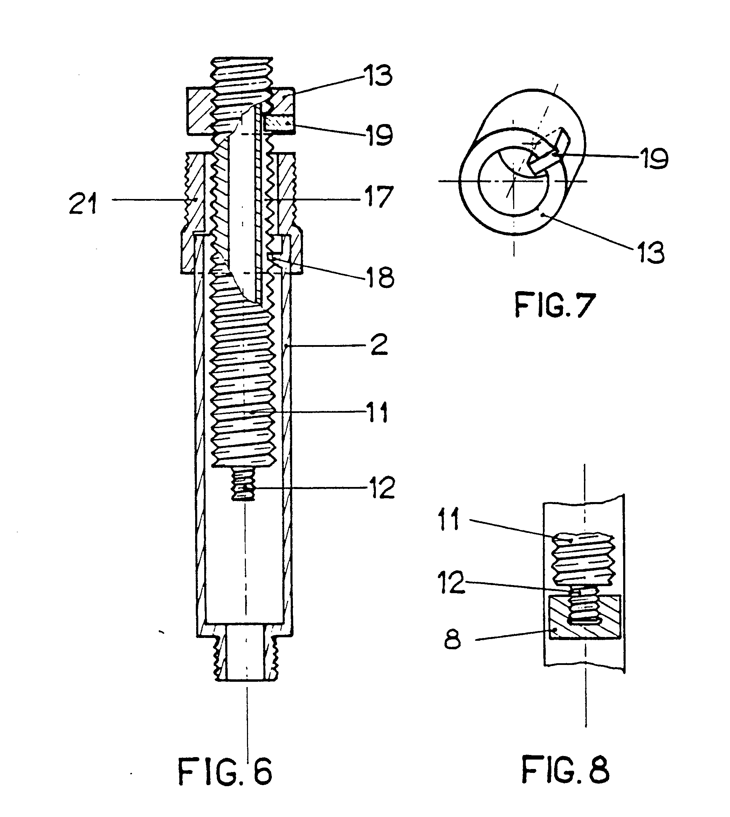

La bague de butée 13 peut être placée : .
- ou bien près de la partie aval du poussoir fileté 11 (cas des figures 1 à 5),
- ou bien, au contraire, au sommet du stylo, comme illustré sur les figures 6 et 8.

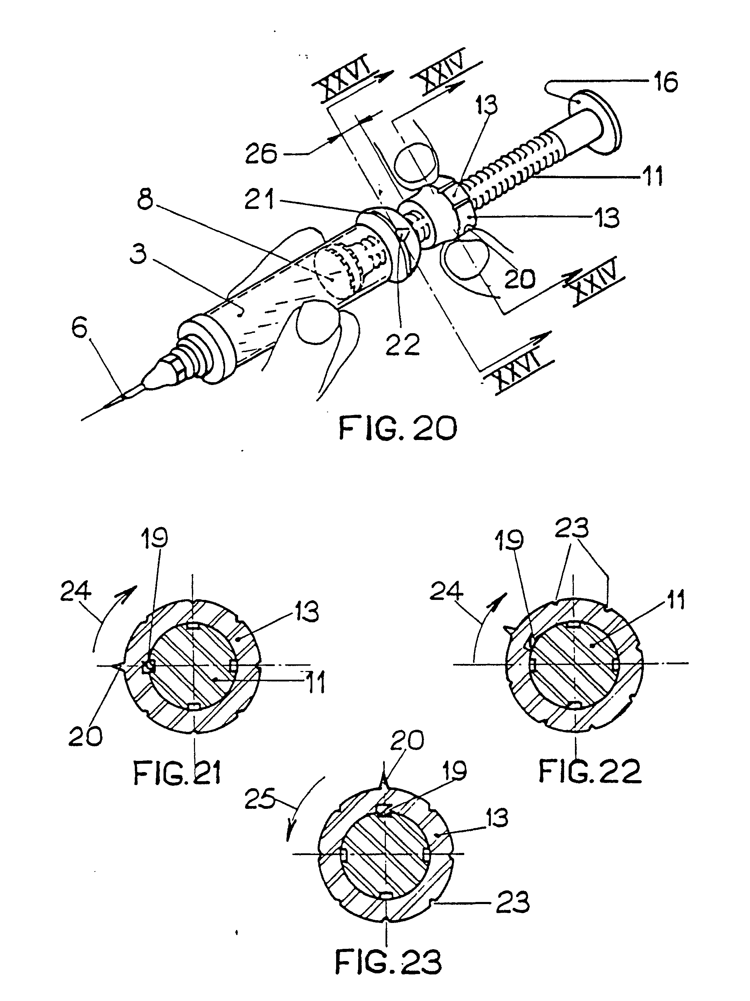

Dans tous les cas, la bague de butée 13 est pourvue d'au moins un cliquet élastique intérieur 19 (figures 21 à 23). De plus, elle co porte un repère extérieur 20, disposé en relief.

Enfin, un repère extérieur 20-22 peut être prévu en relief sur la couronne fixe 21 du corps 2.

Le fonctionnement est le suivant :

On voit, sur les figures 1 à 5, que la présentation de l'aiguille 6, la perforation de la membrane stérile de la carpule 4 par vissage dans l'embout 5, et l'extraction du capuchon d'emballage stérile 7, s'effectuent sans qu'à aucun moment, l'aiguille 6 ne risque d'être contaminée par un quelconque contact non stérile.

Ensuite, il suffit à l'utilisateur de dévisser la bague 13 et/ou le manchon-poussoir 16-15 d'une quantité repérée qui correspond exactement à la dose de produit que doit injecter le piston 8. Ce dévissage est repéré exactement:
- d'une part, visuellement, par simple observation du décalage des repères 20 (sur la bague 13) et 22 (sur la couronne 21);
- d'autre part, de façon tactile et auditive, par perception de chaque passage sur une rainure 17 du cliquet élastique 19 dont est pourvue la paroi interne de la bague 13.

On peut améliorer encore la précision du repérage tactile ou visuel, en répartissant régulièrement huit encoches 23 sur la périphérie dela bague 13.

On voit que le cliquet 19 permet de librement dévisser la bague 13 sur la tige filetée 11 (rotation dans le sens de la flèche 24), alors qu'il n'est pas possible de( la visser (flèche 25, figure 23).

La bague 13 étant ainsi immobilisée sur la tige 11, à une distance 26 exactement calibrée (figure 20), il suffit d'enfoncer le poussoir 16, par une pression du pouce, pour injecter très exactement la dose voulue du produit contenu dans la carpule 4. L'injection se poursuit jusqu'à ce que la bague 13 vienne à nouveau buter contre la couronne 21, ou contre la face de butée transversale 14. La seringue est alors prête pour une autre injection à pratiquer ultérieurement par un nouveau réglage de la bague 13, qu'on dévisse comme indiqué sur les figures 21 et 22.

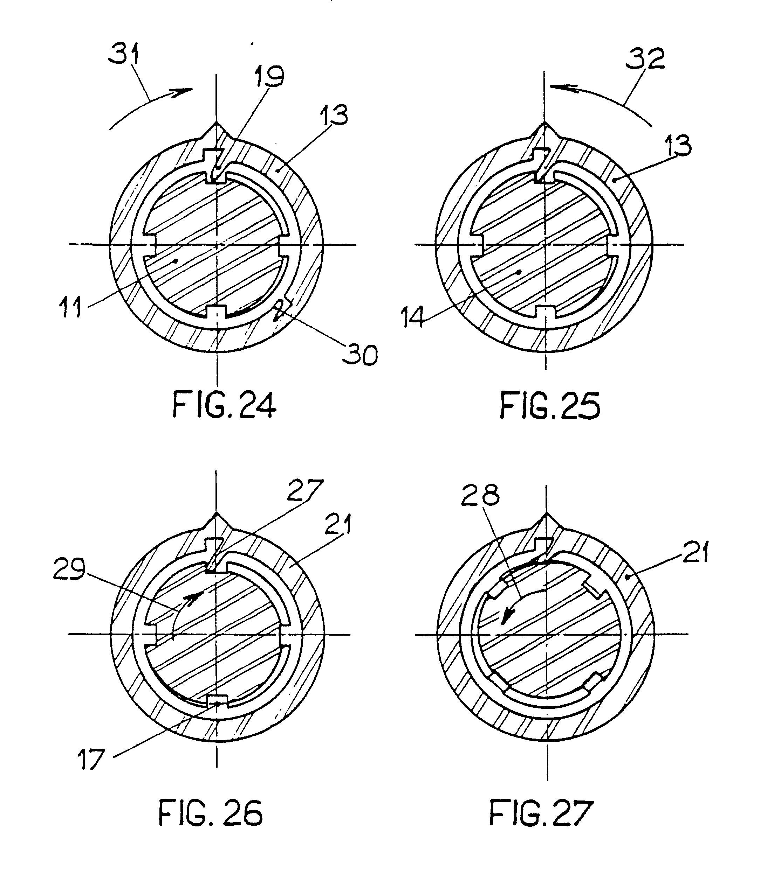

Dans la variante des figures 24 à 27, l'intérieur de la couronne 21 est, lui aussi,fileté et muni d'au moins un cliquet 27, qui coopère avec les rainures 17. Ce cliquet 27 est orienté de façon à permettre librement le vissage de la tige du poussoir 11 dans le sens de la flèche 28 (figure 27), qui provoque l'enfoncement du piston 8 dans la carpule 4. Au contraire, il interdit tout recul du piston 8 (dévissage dans le sens de la flèche 29), (figure 26).

En ce qui concerne la bague 13, on l'a équipée de deux cliquets intérieurs 19 et 30, décalés angulairement d'un nombre impair de huitièmes de tour |(2n + 1) . 45 degrés|, où n est un nombre entier compris entre 0 et 3 inclus). Ainsi, à chaque huitième de tour dans le sens où il dévisse la bague 13 (figure 24, flèche 31), l'utilisateur perçoit le passage d'un des cliquets 19 ou 30. Par contre, toute rotation en sens inverse (figure 25, flèche 32), demeure interdite.

Le fonctionnement est le suivant :

Le réglage s'effectue comme précédemment par dévissage de la bague 13 (flèche 31), jusqu'à définir exactement la distance 26 voulue. Ce réglage s'effectue par contrôle visuel, tactile et/ou auditif, en comptant les crans, dont chacun correspond à un huitième de tour. Une fois le réglage effectué, il suffit de visser la tige filetée du poussoir 11 dans la couronne filetée 21 (figure 27, flèche 28) pour provoquer l'injection jusqu 'à ce que la bague 13 vienne à nouveau en butée sur la couronne 21.

Le vissage de la tige filetée du poussoir 11 peut être effectué :

- soit en agissant sur la tête moletée de la tige du poussoir 11;
- soit en faisant tourner la bague 13 dans le sens de la flèche 32 où elle entraîne avec elle la tige filetée du poussoir 11.

L'invention comporte en particulier les avantages suivants:

- réglage facile de chaque dose à injecter (distance 26), même pour un aveugle ou un sourd;
- impossibilité d'effectuer des fausses manoeuvres;
- à la fin d'une injection, la seringue est automatiquement prête pour l'injection suivante.

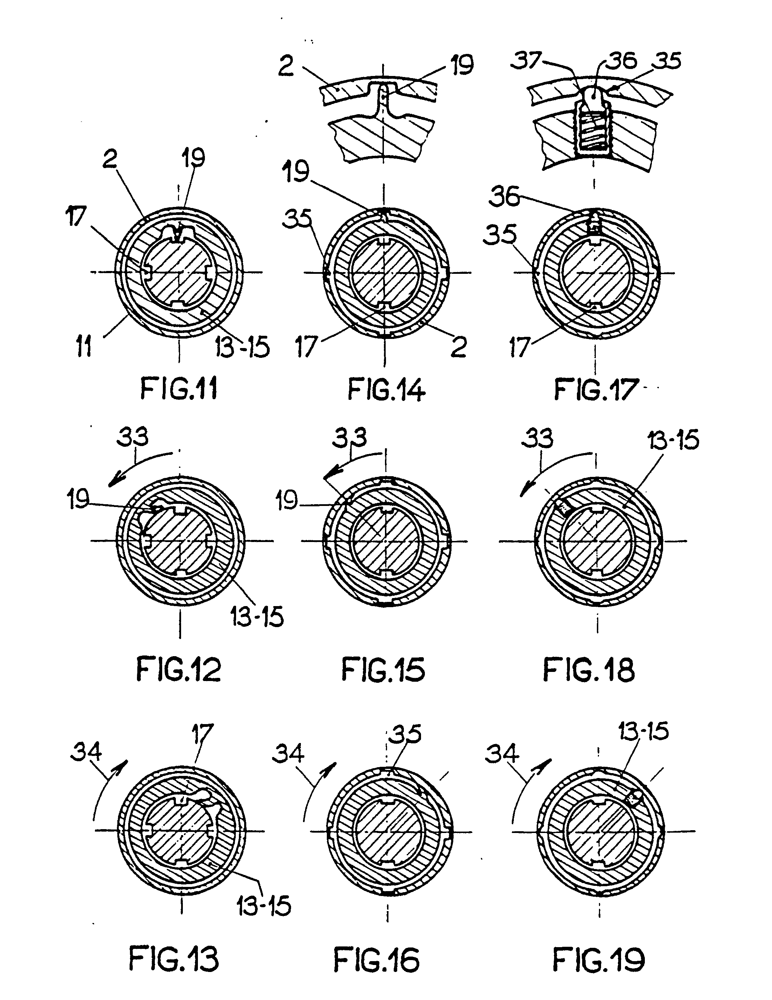

Dans l'exemple illustré sur les figues 11 à 13, la bague de butée 13 est solidaire du manchon 15, qui entoure la tige filetée du poussoir 11. L'ensemble est entouré par le corps 2 du stylo. Le fonctionnement du cliquet 19 reste analogue à celui qui vient d'être décrit avec référence aux figures 24 et 25. En particulier, on voit, sur la figure 12, la position occupée par le cliquet 19, pendant que l'utilisateur est en train d'augmenter le dosage à injecter : pour cela, il fait tourner le manchon-poussoir 13-15 dans le sens indiqué par la flèche 33. Au contraire, lorsqu'il désire diminuer le volume de la dose à injecter, il lui suffit de faire tourner le manchon-poussoir 13-15 dans le sens indiqué sur la figure 13, par la flèche 34.

Dans la variante des figures 14 à 16, le cliquet 19 équipe la face extérieure de l'ensemble manchon-poussoir 13,15, tandis que des rainures longitudinales 35 sont creusées dans la face interne du corps 2. Ici encore, la rotation de l'ensemble manchon-bague de butée 13,15 dans le sens de la flèche 33 (figure 15) va dans le sens d'une diminution de la quantité dosée, alors qu'une rotation en sens inverse (flèche 34, figure 16), correspond à une augmentation du dosage.

Une autre variante possible a été représentée sur les figures 17 à 19, à savoir le remplacement du cliquet 19 par un doigt d'arrêt 36, escamotable à l'encontre de moyens de rappel élastique 37. Ces derniers peuvent être constitués, par exemple, par un bloc de caoutchouc, ou par un ressort métallique.

Des encoches 35 sont comme précédemment, prévues face au doigt d'arrêt escamotable 36. Ici encore, la rotation de l'ensemble manchon-poussoir 13-15 dans le sens de la flèche 33 (figure 18) correspond à une augmentation du dosage; la rotation dans le sens de la flèche 34 (figure 19) entraîne une diminution du dosage.

Pour augmenter encore la précision du dosage, tout en garantissant une totale sécurité d'utilisation, on prévoir préférablement, sur le poussoir fileté 11 qui va être décrit, un système à ailettes anti-retour telles que représentées sur les figures 4, 5, 9 et 10.

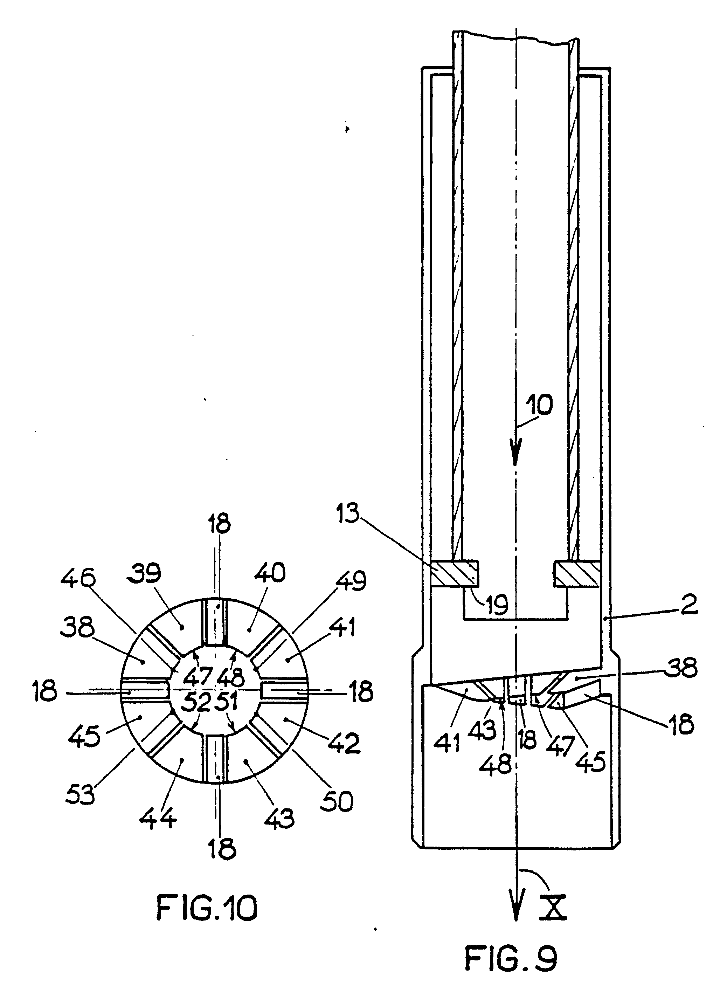

Des ailettes solidaires de la paroi intérieure du corps 2 sont prévues suivant une disposition qui les fait converger vers le centre en direction de l'extrémité aval du dispositif.La partie arrière de chacune de ces ailettes flexibles est solidaire de la paroi interne du corps 2. Par contre, chacune d'elles se termine en direction du centre, par un bord 46 à 53. Chacun de ces bords constitue l'un des segments successifs d'une ligne répartie autour de la tige filetée du poussoir 11. Tous ces bords 46 à 53 sont appliqués à force contre la paroi extérieure filetée du poussoir 11, ce qui entraîne simultanément le fléchissement de toutes les ailettes 38 à 45. L'une au moins d'entre elles voit son bord 46 ou 47 ou 48, etc., ... ou 53, s'engager, par rappel élastique, dans le filetage du poussoir 11. Autrement dit, si les ailettes 38 à 45 sont au nombre de huit (cas de la figure 10) et si le pas du filetage du poussoir 11 est de 1 millimètre, on comprend que chaque enfoncement du poussoir 11 dans le sens de la flèche 10 s'effectuera par crans successifs de 1/8ème de millimètre. Du fait de l'inclinaison du plan général de chaque ailette à la fois vers le centre du corps 2 et vers son extrémité aval, le fléchissement et le clipsage élastique des ailettes 38 à 53 autorisent l'enfoncement du poussoir 11 (flèche 10), mais interdisent son retour en sens inverse. Dans l'exemple numérique qui vient d'être choisi, cet enfoncement et son immobilisation anti-retour s'effectuent donc par crans successifs de 1/8ème de millimètre.

On comprend qu'en multipliant le nombre des ailettes et en réduisant le pas du filetage du poussoir 11, on puisse arriver à une précision extrême dans le dosage. Los de l'enfoncement du poussoir (flèche 10), chaque cran est perçu par l'utilisateur à la fois au toucher et à l'audition, lorsque l'ailette échappe alors que la suivante s'encliquète sur le filetage. Il suffit donc à l'utilisateur de compter les déclics, pour atteindre une précision de l'ordre du centième de millimètre. Cette précision permet, par exemple, à un diabétique d'effectuer un grand nombre d'injections successives à partir d'une seule recharge ou carpule 4.

En ce qui concerne le réarmement de l'appareil après chaque injection, deux variantes sont possibles.

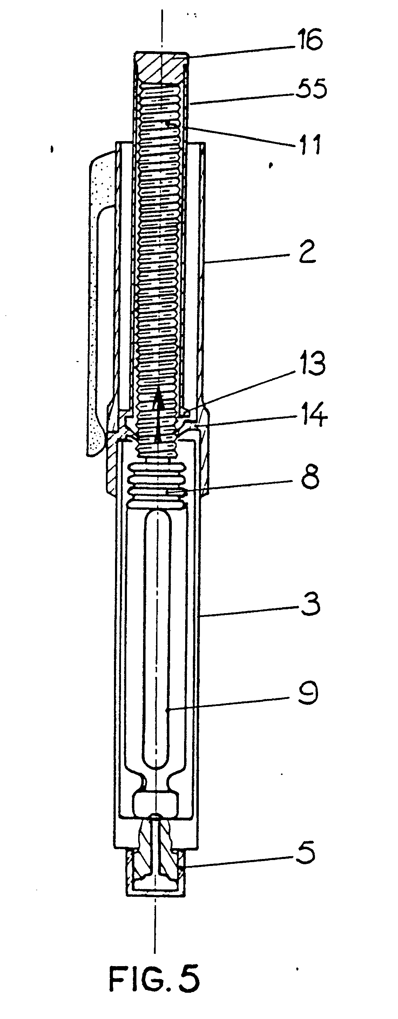

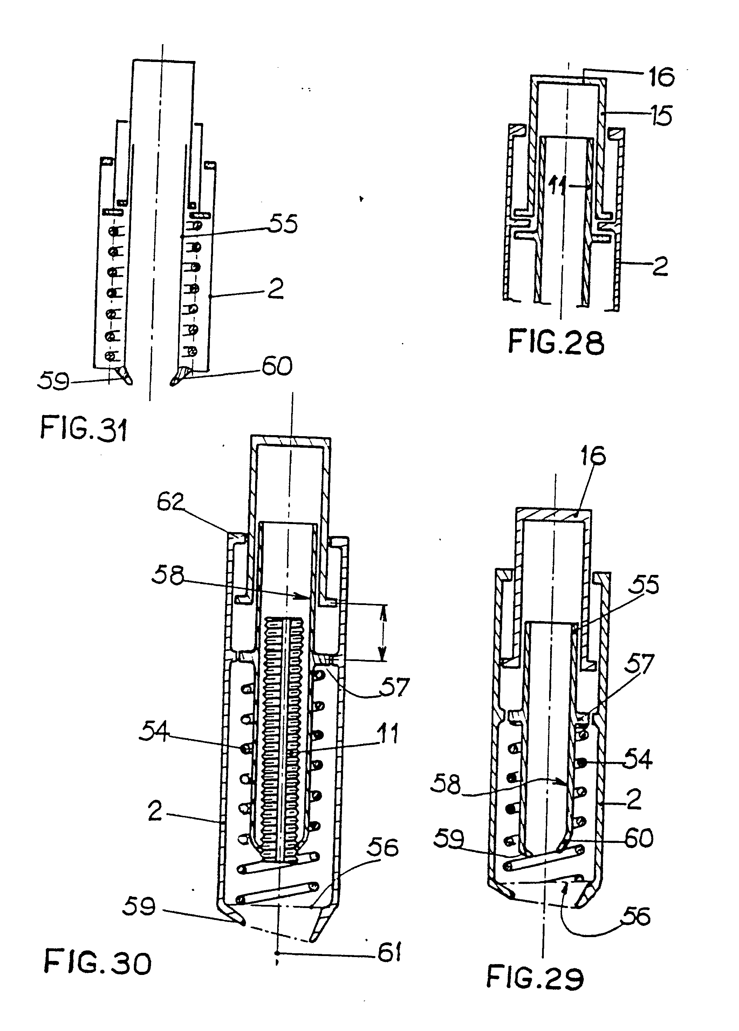

Dans le cas des figures 5 et 28, le réarmement s'effectue manuellement. Pour cela, le manchon 15 est vissé sur le poussoir 11, dont il est solidaire dans le sens longitudinal, comme un écrou sur une vis :
en utilisation, le bouton poussoir 15, 16 ne peut donc être ramené en arrière (sens inverse de la flèche 10, figure 9), que par une opération manuelle.

Au contraire, on a représenté sur les figures 4, et 29 à 31, un système de réarmement automatique après chaque injection. Pour cela, on prévoit un ressort hélicoïdal de compression 54, logé autour du manchon poussoir désigné ici par la référence 55. Ce ressort 54 est logé dans l'espace intermédiaire défini entre la périphérie du manchon 55, et l'intérieur du corps fixe 2. Il prend appui :
- en bas, sur un épaulement transversal annulaire 56 défini à l'intérieur du corps 2, au-dessus des ailettes anti-retour 38 à 45;
- en haut, sur une collerette transversale 57, prévue en relief autour du manchon poussoir 55. Par ailleurs, la paroi interne 58 du manchon poussoir 55 est lisse, si bien qu'elle coulisse librement autour de la tige filetée du poussoir 11 qu'elle contient.

A sa partie inférieure, le manchon poussoir 55 comporte des ailettes flexibles anti-retour 59, 60 du genre précité: comme les ailettes 38 à 45, elles convergent vers l'axe géométrique central 61 du dispositif, en direction de son extrémité aval. Sous l'effet de leur propre flexibilité, elles viennent s'encliqueter dans les filets du poussoir fileté 11 où elles jouent un rôle anti-recul. En d'autres termes :
- si l'on appuie sur le poussoir 55-16 dans le sens de la flèche 10 (figure 4), le poussoir 11 est solidaire du bouton 16-55 avec lequel il s'enfonce : cela permet de procéder à l'injection d'une dose;
- par contre, lorsqu'on relâche le bouton 16-55, il est rappelé à sa position de départ par coulissement dans le sens inverse de la flèche 10, ceci sous la poussée élastique du ressort 54; pendant ce temps, le poussoir 11 reste immobilisé par les ailettES 38 à 45 qui lui interdisent tout déplacement dans ce sens, la remontée du manchon 55 à l'encontre de la flèche 10 s'effectuant alors par effacements successifs des ailettes 59, 60 qui se déforment élastiquement en remontant le long du filetage du poussoir 11.

On voit que, grâce à ce dispositif, le bouton poussoir 16,55 après chaque injection, automatiquement à la position de repos illustrée sur les figures 4 et 31, où l'épaulement annulaire 57 du manchon 55 vient en butée contre un bourrelet annulaire interne 62 du corps fixe 2.

On voit que la référence 63 représente la course maxima de compression du ressort 54.

## Revendications

1.- Dispositif de délivrance de doses multiples contrôlées, comprenant :
- un corps creux allongé (2) propre à loger intérieurement une recharge (4) d'un produit liquide, actionnée par poussée (8), ainsi qu'à recevoir du côté aval de la recharge (4) un embout de délivrance (6);
- un poussoir (11,15,55) monté à coulissement dansle corps (2), du côté amont de la recharge, et
- des moyens limiteurs (13,14) de l'excursion permise au poussoir (11,15,55) vers la recharge (4), caractérisé en ce qu'il comprend un moyen anti-retour constitué d'au moins une ailette flexible (38-45,59,60) agissant au niveau du poussoir (11,15,55) et du corps (2).

2.- Dispositif suivant la revendication 1, caractérisé en ce que le poussoir comprend une tige de poussée (11), la ou les ailettes (38-45) formant anti-retour vers l'amont pour la tige de poussée par rapport au corps (2).

3.- Dispositif suivant la revendication 2, caractérisé en ce que le poussoir comprend un manchon (15,55) qui coopère avec la tige de poussée (11).

4.- Dispositif suivant la revendication 3, caractérisé en ce que le manchon (15,55) coopère avec la tige de poussée (11) également par l'intermédiaire d'au moins une ailette (59,60) formant anti-retour vers l'aval.

5.- Dispositif suivant l'une des revendications 3 et 4, caractérisé en ce qu'il comporte un rappel élastique du manchon (55) vers l'amont.

6.- Dispositif suivant l'une des revendications 1 à 5, caractérisé en ce que la ou les ailettes (38-45) sont prévues suivant une disposition qui les fait converger vers le centre en direction de l'embout du dispositif (1).

7.- Dispositif suivant l'une des revendications 1 à 6, caractérisé en ce que la ou les ailettes (38-45,59,60) coopèrent avec des crans ou des sections de filetage.

8.- Dispositif suivant l'une des revendications 1 à 7, caractérisé en ce que la ou les ailettes (38-45,59,60) définissent sensiblement la direction d'un plan incliné à la fois vers l'axe longitudinal (61) et en direction de l'extrémité aval du dispositif.

9.- Dispositif suivant l'une des revendications 1 à 8, caractérisé en ce que le poussoir (11) est muni au moins en partie de sections de filetage périphérique.

10.- Dispositif suivant l'une des revendications précédentes, caractérisé en ce qu'il comporte des moyens anti-rotation du poussoir par coopération d'au moins une rainure longitudinale (17) et d'un ergot (18).

11.- Dispositif suivant la revendication 10, caractérisé en ce que les moyens anti-rotation comprennent plusieurs rainures longitudinales (17), régulièrement distribuées sur la périphérie du poussoir (11).

12.- Dispositif suivant l'une des revendications précédentes, caractérisé en ce que les moyens limiteurs comprennent une bague (13) réglable en position sur le poussoir (11), coopérant avec une butée (14) faisant saillie à l'intérieur du corps.

13.- Dispositif suivant la revendication 12, caractérisé en ce que la bague (13) coopère angulairement et axialement à la façon d'un écrou réglable avec un filetage périphérique extérieur du poussoir (11).

14.- Dispositif suivant l'une des revendications 12 et 13, caractérisé en ce que la bague (13) comporte, sur sa périphérie, des moyens crantés effaçables élastiquement (doigt élastique (36)-encoches (35)), pour assurer cran par cran son positionnement angulaire sur le poussoir fileté (11).

15.- Dispositif suivant la revendication 14, caractérisé en ce que les moyens crantés forment anti-retour angulaire (cliquet (19)-rainure (17)).

16.- Dispositif suivant l'une des revendications 12 à 15, caractérisé en ce que la bague (13) est définie par un manchon (15) mobile le long d'une tige de poussée (11) du poussoir, avec anti-retour vers l'aval.

17.- Dispositif suivant l'une des revendications 1 à 9 et 12 à 16, caractérisé en ce qu'il comporte des moyens crantés effaçables élastiquement formant anti-retour angulaire (cliquet (27)-rainures (17)) entre le poussoir (11,15,55) et le corps (2).

18.- Dispositif suivant la revendication 17, caractérisé en ce que le cliquet (19) équipe la face extérieure de l'ensemble manchon-poussoir (11,13,15) tandis que des rainures longitudinales (35) sont creusées en correspondance dans la face interne du corps (2).

FIG.1

FIG. 2

16

2

1

4

10

8

3

9

6

7

FIG.3

FIG. 4

FIG.5

FIG. 6

FIG.7

FIG. 8

FIG.10

FIG.9

EP 0 416 975 A1

FIG.11

FIG.14

FIG.17

FIG.12

FIG.15

FIG.18

FIG.13

FIG.16

FIG.19

13

FIG. 20

FIG. 21

FIG. 22

FIG. 23

FIG. 24

FIG. 25

FIG. 26

FIG. 27

FIG.31

FIG.28

FIG.30

FIG.29

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | FR-A-2 612 782 (A. TADDEI)<br>* abrégé; page 1, lignes 7-14; pages 3-5; revendications 1,2,7,8; figures 5-11,13-16 * | 1,10,11 | A 61 M 5/315<br>G 01 F 11/02 |
| A | EP-A-0 268 191 (WILHELM HASELMEIER GMBH & CO.)<br>* abrégé; figures 1,3,5 * | 1 | |
| A | EP-A-0 293 572 (D.C.P. AF 1988 A/S)<br>* abrégé; revendication 1; figures 2-4 * | 1 | |

DOMAINES TECHNIQUES RECHERCHES (Int. Cl.5)

A 61 M
G 01 F

Le présent rapport de recherche a été établi pour toutes les revendications

| Lieu de la recherche | Date d'achèvement de la recherche | Examinateur |
|---|---|---|
| Berlin | 27 novembre 90 | VORROPOULOS G |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention

E : document de brevet antérieur, mais publié à la date de dépôt ou après cette date
D : cité dans la demande
L : cité pour d'autres raisons

& : membre de la même famille, document correspondant